# EUROPÄISCHE PATENTSCHRIFT

(11) **EP 0 864 326 B1**
(45) Veröffentlichungstag und Bekanntmachung des Hinweises auf die Patenterteilung: **02.06.2004**
(21) Anmeldenummer: 98104478.7
(22) Anmeldetag: 12.03.1998
(51) Int. Cl.: A61K 9/26, A61K 9/20

(54) **Mehrphasige wirkstoffhaltige Zubereitungsformen**
Multiphasic preparation comprising an active agent
Préparation multiphasique comprenant un agent actif

(30) Priorität: 12.03.1997 DE 19710009
(43) Veröffentlichungstag der Anmeldung: 16.09.1998
(73) Patentinhaber: Abbott GmbH & Co. KG, 65205 Wiesbaden (DE)
(72) Erfinder: Rosenberg, Joerg, Dr., 67158 Ellerstadt (DE); Zeidler, Jürgen, Dr., 67112 Mutterstadt (DE); Breitenbach, Jörg, Dr., 68199 Mannheim (DE); Berndl, Gunther, Dr., 67273 Herxheim (DE); Kleinke, Andreas, Dr., 67063 Ludwigshafen (DE)
(74) Vertreter: Kinzebach, Werner, Dr.

(56) Entgegenhaltungen:
- EP-A- 0 596 203
- EP-A- 0 598 606
- WO-A-93/20138
- WO-A-96/25149
- WO-A-96/29060
- WO-A-97/02017
- DE-A- 2 627 533
- DE-A- 3 520 184

## Beschreibung

Die vorliegende Erfindung betrifft ein Verfahren zur Herstellung einer festen oder halbfesten, mindestens zweiphasigen wirkstoffhaltigen Zubereitungsform sowie eine nach diesem Verfahren erhältliche Zubereitungsform.

Ein häufig auftretendes Problem in der pharmazeutischen Technologie ist das Einbringen von untereinander unverträglichem Wirkstoff in eine Arzneiform.

Nach dem Stand der Technik wird dies durch die Herstellung von Mantel- oder Mehrschichtentabletten gelöst. Solche Tablettenformen erlauben nicht nur die Trennung von unverträglichen Wirkstoffen, sondern auch die Trennung von Initial- und Erhaltungsdosis bei Retardarzneiformen. Diese Arzneiformen werden üblicherweise durch konventionelles Verpressen erhalten. Dies erfordert jedoch speziell konstruierte Tablettenpressen und mindestens zwei Füll- und Preßstationen (vgl. "Pharmazeutische Technologie", Georg Thieme Verlag, 4. Auflage, 1993, Seite 300 ff.). Dieses Verfahren ist jedoch aufwendig und kostenintensiv.

Weiterhin ist es bekannt, durch Komprimieren eines Gemisches unterschiedlich vorbehandelter Granulate Mischgranulat-Tabletten herzustellen. Dabei werden beispielsweise unbehandelte Arzneistoffe (Initialdosis) neben mit Fetten umhüllten oder mit Lack überzogenen Arzneistoffen (Erhaltungsdosis) verarbeitet (vgl. R. Voigt, Lehrbuch der pharmazeutischen Technologie, 1987, S. 225). Auch dieses Verfahren ist relativ aufwendig.

Aus der EP-A 580 860 ist bekannt, daß Wasser oder Lösungsmittel während des Extruksionsherganges zudosiert werden können. Eine Zudosierung von Wirkstoffen ist jedoch nicht beschrieben.

Die WO 97/02017 beschreibt eine oral verabreichbare Formulierung zur kontrollierten Freisetzung eines schwer wasserlöslichen Wirkstoffes. Dieser liegt in Form einer festen Dispersion in einem hydrophilen Poloxamer-Polymer vor. Die DE 35 20 184 beschreibt feste Dispersionen, bei denen ein Wirkstoff in einer Matrix aus Polyethylenglykol enthalten ist. Mindestens 5 Gew.-% der Wirkstoffteilchen bilden eine zusammenhängende, poröse Sekundärstruktur, in welche weitere Wirkstoffteilchen mit einem Durchmesser bis zu 100 µm eingeschlossen sind. Die WO 96/29060, WO 96/25149 und EP 596 203 beschreiben Zubereitungen, die durch Schmelzextrusion hergestellt werden. Die WO 93/20138 beschreibt ein Polymersystem, das eine teilweise lösliche Verbindung sowohl in gelöster als auch dispergierter Form enthält.

Der Erfindung lag die Aufgabe zugrunde, ein einfaches Verfahren zur Herstellung von wirkstoffhaltigen Formen zu finden, welches das Einbringen untereinander unverträglicher Wirkstoffe oder unterschiedlich freisetzender wirkstoffe in eine Arzneiform oder die Herstellung von Formen mit mehrphasigem Freisetzungsverhalten erlaubt.

Demgemäß wurde ein Verfahren zur Herstellung einer festen oder halbfesten, mindestens zweiphasigen wirkstoffhaltigen Zubereitungsform gefunden, wobei eine Phase eine Matrix bildet, die mindesten einen Wirkstoff in Form einer festen Lösung enthält, und mindestens eine weitere Phase multipartikulär in Form von Partikeln, die mindestens einen Wirkstoff und ein Additiv umfassen, in die Matrix eingebettet ist, wobei die Matrix eine Komponente umfasst, die ausgewählt ist unter unvemetzten Homo- oder Copolymeren des N-Vinylpyrrolidons mit K-Werten nach Fickentscher von 12 bis 100, Hydroxyalkylcellulosen, Celluloseestern, Acrylat- oder Methacrylat-haltigen Polymeren und Polyethylenglykolen, wobei die Polyethylenglykole ein Molekulargewicht im Bereich von 1000 bis 20 000 aufweisen, wobei man eine Mischung der Komponenten der Matrixphase durch Energieeintrag plastifiziert, während oder nach der Plastifizierung die mindestens eine weitere wirkstoff- und additivhaltige Phase in Form von Partikeln mit einer Größe von 0,5 bis 3 mm homogen in die Matrixphase einbettet und die noch plastische Masse anschließend formt.

Partikel können erfindungsgemäß Granulate, Pellets oder Kristallpartikel sein, wobei die Kristallpartikel vorzugsweise beschichtet sind.

Die Partikel können auf herkömmliche Weise erhalten werden, so beispielsweise durch Feuchtgranulation eines oder mehrerer Wirkstoffe mit üblichen Additiven. Granulate können auch in an sich bekannter Weise durch Schmelzextrusion einer wirkstoffhaltigen Polymerschmelze und anschließender Formgebung durch Heiß- oder Kaltabschlag, Prill- oder Vertropfungsverfahren erhalten werden. Auch die Herstellung von Pellets kann nach an sich üblichen Verfahren erfolgen, beispielsweise durch Trockengranulation. Auch beschichtete oder unbeschichtete Wirkstoffkristalle können nach für den Fachmann bekannten Verfahren erhalten werden. Die Partikelgröße ist nicht kritisch. Um eine gute Handhabung der Partikel zu erhalten, empfiehlt es sich, Partikel in der Größenordnung von 0,5 bis 3 mm, vorzugsweise 0,5 bis 2 mm zu verwenden.

Partikel im Sinne dieser Erfindung sind auch Mikrotabletten. Die Herstellung von Mikrotabletten kann ebenfalls auf an sich bekannte Weise erfolgen.

Die Art der Additive richtet sich vor allem danach, welche Freisetzungsrate für die partikuläre Phase gewünscht wird, also danach, ob die partikuläre Phase schnell oder langsam freisetzend sein soll.

Bei Verwendung von beschichteten Wirkstoffkristallen kann der Überzug rein stabilisierende oder auch retardierende Eigenschaften aufweisen. Es können auch Überzüge eingesetzt werden, die sich in bestimmten pH-Bereichen auflösen.

Auf diese Weise können Kombinationsarzneiformen erhalten werden, in denen der oder die Wirkstoffe aus den partikulären Anteilen an unterschiedlichen Stellen des Verdauungstrakts freigesetzt werden können. Solche Überzüge können beispielsweise Polyacrylate oder Methacrylsäure-Copolymere (Eudragit-Typen) sein.

Der Anteil an partikulär zugesetztem Aliquot kann 0,01 bis 90 Gew.-%, bevorzugt 0,1 bis 70 Gew.-%, besonders bevorzugt 0,5 bis 50 Gew.-% betragen, bezogen auf das Gesamtgewicht der fertigen wirkstoffhaltigen Form.

In der erfindungsgemäßen Zubereitungsform liegen die Partikel multipartikulär in einer anderen Phase eingebettet vor. Die andere Phase bildet also die Matrix für die partikuläre Phase.

Prinzipiell sind alle schmelzbaren oder erweichbaren Substanzen als Aufbaustoffe für die Matrixphase einsetzbar, sofern sie sich unter den Verarbeitungsbedingungen nicht zersetzen. Die erforderliche Thermoplastizität kann auch durch Zusatz geeigneter Hilfsmittel herbeigeführt werden.

Geeignete Aufbaustoffe für die Matrix sind thermoplastisch verarbeitbare polymere Bindemittel. Als solche kommen unvernetzte Homo- oder Copolymere des N-Vinylpyrrolidons mit K-Werten nach Fikentscher von 12 bis 120, bevorzugt 20 bis 100 in Betracht, wobei als Comonomere vorzugsweise Vinylester wie beispielsweise Vinylpropionat, Vinylbutyrat oder, insbesondere Vinylacetat in Betracht kommen, oder aber auch N-Vinylimidazol oder N-Vinylcaprolactam.

Geeignete Bindemittel sind weiterhin Hydroxyalkylcellulosen wie Hydroxypropylcellulose, und Celluloseester wie Celluloseacetat, Celpropylcellulose, und celluloseester wie Celluloseacetat, Cellulosephthalat, Celluloseacetatpropionat, Celluloseacetatphthalat oder ähnliche. Ebenfalls geeignete Bindemittel sind Acrylat- oder Methacrylat-haltige Polymerisate, beispielsweise Eudragit-Typen.

Geeignete Matrixpolymere sind auch Polyethylenglykole mit Molekulargewichten im Bereich von 1000 bis 20000.

Weiterhin können in die Matrix übliche physiologisch verträgliche Hilfsstoffe eingearbeitet werden, beispielsweise Füllstoffe, Schmiermittel, Formentrennmittel, Weichmacher, Triebmittel, Stabilisatoren, Farbstoffe, Aromastoffe oder Fliessmittel.

Beispiele für Füllstoffe sind anorganische Füllstoffe wie die Oxide von Magnesium, Aluminium, Silizium, Titan etc. in einer Konzentration von 0,01 bis 50, vorzugsweise von 0,20 bis 20 Gew.-% bezogen auf das Gesamtgewicht der Arzneiform.

Beispiele für Schmiermittel sind Stearate von Aluminium, Calcium und Magnesium sowie Talkum und Silicone in einer Konzentration von 0,1 bis 5, vorzugsweise von 0,1 bis 3 Gew.-% bezogen auf das Gesamtgewicht der Form.

Als Zerfallsbeschleuniger können z.B. Natriumcarboxymethylstärke, Crospovidon eingesetzt werden. Auch Benetzungsmittel wie Natriumlaurylsulfat, Natriumdocusat sind einsetzbar. Weiterhin können Salze wie Na₂CO₃ oder NaHCO₃ eingesetzt werden.

Beispiele für Weichmacher beinhalten niedermolekulare Poly(alkylenoxide), wie z.B. Poly(ethylenglycole), Poly(propylenglycole), Poly(ethylen-propylenglycole); organische Weichmacher mit niederem Molekulargewicht wie Glycerin, Pentaerythrit, Glycerinmonoacetat, Diacetat oder Triacetat, Propylenglycol, Natriumdiethylsulfosuccinat und ähnliche Verbindungen zugefügt in Konzentrationen von 0,5 bis 15, vorzugsweise von 0,5 bis 5 Gew.-% bezogen auf das Gesamtgewicht der Arzneiform.

Durch unterschiedliches Anfärben der verschiedenen Phasen lassen sich die Formen auch ansprechend und mit hohem Wiedererkennungswert gestalten. Beispiele für Farbstoffe sind bekannte Azofarbstoffe, organische und anorganische Pigmente, oder Farbmittel natürlicher Herkunft. Anorganische Pigmente sind bevorzugt, in Konzentrationen von 0,001 bis 10, vorzugsweise von 0,5 bis 3 Gew.-% bezogen auf das Gesamtgewicht der Arzneiform.

Geschmacksstoffe und Aromen wie beispielsweise Vanillin sind bevorzugt in der Trägermatrix enthalten.

Darüberhinaus können noch andere Additive zugefügt werden, die die Fließeigenschaften der Mischung verbessern oder als Formtrennmittel wirken, wie z.B. tierische oder pflanzliche Fette, bevorzugt in ihrer hydrierten Form, besonders solche, die bei Raumtemperatur fest sind. Diese Fette haben vorzugsweise einen Schmelzpunkt von 50°C oder höher. Bevorzugt sind Triglyceride der C₁₂-, C₁₄-, C₁₆- und C₁₈-Fettsäuren. Die gleiche Funktion können auch Wachse wie z.B. Carnaubawachs erfüllen. Diese Additive können alleine ohne Zusatz von Füllstoffen oder Weichmachern zugesetzt werden. Diese Fette und Wachse können vorteilhaft alleine oder zusammen mit Mono- und/oder Diglyceriden oder Phosphatiden, besonders Lecithin, beigemischt werden. Die Mono- und Diglyceride stammen vorzugsweise von den oben beschriebenen Fett-Typen ab, d.h. C₁₂-, C₁₄-, C₁₆- und C₁₈-Fettsäuren. Die Gesamtmenge an Fetten, Wachsen, Mono-, Diglyceriden und/oder Lecithinen beträgt 0,1 bis 30, vorzugsweise 0,1 bis 5 Gew.-% bezogen auf das Gesamtgewicht der Arzneiform.

Als Fließregulierungsmittel können z.B. Aerosile oder Talkum Verwendung finden.

Ferner können auch Stabilisatoren zugefügt werden, wie z.B. Antioxidantien, Lichtstabilisatoren, Hydroperoxid-Vernichter, Radikalfänger und Stabilisatoren gegen mikrobiellen Befall.

Unter Hilfsstoffen im Sinne der Erfindung sind auch Substanzen zur Herstellung einer festen Lösung mit dem pharmazeutischen Wirkstoff zu verstehen. Diese Hilfsstoffe sind beispielsweise Pentaerythrit und Pentaerythrit-tetraacetat, Polymere wie z.B. Polyethylen- bzw. Polypropylenoxide und deren Blockcopolymere (Poloxamere), Phosphatide wie Lecithin, Homo- und Copolymere des Vinylpyrrolidons, Tenside wie Polyoxyethylen-40-stearat sowie Zitronen- und Bernsteinsäure, Gallensäuren, Sterine und andere wie z.B. bei J.L. Ford, Pharm. Acta Helv. 61, 69-88 (1986) angegeben.

Als pharmazeutische Hilfsstoffe gelten auch Zusätze von Basen oder Säuren zur Steuerung der Löslichkeit eines Wirkstoffes (siehe z.B. K. Thoma et al., Pharm. Ind. 51, 98-101 (1989)).

Als Wirkstoffe im Sinne dieser Erfindung eignen sich grundsätzlich alle Wirkstoffe, die sich unter den Verarbeitungsbedingungen nicht zersetzen.

Das erfindungsgemäße Verfahren ist beispielsweise zur Verarbeitung der folgenden Wirkstoffe geeignet:

Acebutolol, Acetylcystein, Acetylsalicylsäure, Acyclovir, Albrazolam, Alfacalcidol, Allantoin, Allopurinol, Ambroxol, Amikacin, Amilorid, Aminoessigsäure, Amiodaron, Amitriptylin, Amlodipin, Amoxicillin, Ampicillin, Ascorbinsäure, Aspartam, Astemizol, Atenolol, Beclomethason, Benserazid, Benzalkonium-Hydrochlorid, Benzocain, Benzoesäure, Betamethason, Bezafibrat, Biotin, Biperiden, Bisoprolol, Bromazepam, Bromhexin, Bromocriptin, Budesonid, Bufexamac, Buflomedil, Buspiron, Coffein, Campher, Captopril, Carbamazepin, Carbidopa, Carboplatin, Cefachlor, Cefaflexin, Cefatroxil, Cefazolin, Cefixim, Cefotaxim, Ceftazidim, Ceftriaxon, Cefuroxim, Celedilin, Chloramphenicol, Chlorhexidin, Chlorpheniramin, Chlortalidon, Cholin, Cyclosporin, Cilastatin, Cimetidin, Ciprofloxacin, Cisapride, Cisplatin, Clarithromycin, Clävulansäure, Clomibramin, Clonazepam, Clonidin, Clotrimazol, Codein, Cholestyramin, Cromoglycinsäure, Cyanocobalamin, Cyproteron, desogestrel, Dexamethason, Dexpanthenol, Dextromethorphan, Dextropropoxiphen, Diazepam, Diclofenac, Digoxin, Dihydrocodein, Dihydroergotamin, Dihydroergotoxin, Diltiazem, Diphenhydramin, Dipyridamol, Dipyron, Disopyramid, Domperidon, Dopamin, Doxocyclin, Enalapril, Ephedrin, Epinephrin, Ergocalciferol, Ergotamin, Erythromycin, Estradiol, Ethinylestradiol, Etoposid, Eucalyptus Globulus, Famotidin, Felodipin, Fenofibrat, Fenoterol, Fentanyl, Flavin-Mononucleotid, Fluconazol, Flunarizin, Fluorouracil, Fluoxetin, Flurbiprofen, Furosemid, Gallopamil, Gemfibrozil, Gentamicin, Gingko Biloba, Glibenclamid, Glipizid, Clozapin, Glycyrrhiza glabra, Griseofulvin, Guaifenesin, Haloperidol, Heparin, Hyaluronsäure, Hydrochlorothiazid, Hycrocodon, Hydrocortison, Hydromorphon, Ipratropium-Hydroxid, Ibuprofen, Imipenem, Indomethacin, Iohexol, Iopamidol, Isosorbid-Dinitrat, Isosorbid-Mononitrit, Isotretinoin, Ketotifen, Ketoconazol, Ketoprofen, Ketorolac, Labatalon, Lactulose, Lecithin, Levocarnitin, Levodopa, Levoglutamid, Levonorgestrel, Levothyroxin, Lidocain, Lipase, Lipramin, Lisinopril, Loperamid, Lorazepam, Lovastatin, Medroxypregesteron, Menthol, Methotrexat, Methyldopa, Methylprednisolon, Metoclopramid, Metoprolol, Miconazol, Midazolam, Minocyclin, Minoxidil, Misoprostol, Morphin, Multivitamin-Mischungen bzw. -Kombinationen und Mineralsalze, N-Methylephedrin, Naftidrofuryl, Naproxen, Neomycin, Nicardipin, Nicergolin, Nicotinamid, Nicotin, Nicotinsäure, Nifedipin, Nimodipin, Nitrazepam, Nitrendipin, Nizatidin, Norethisteron, Norfloxacin, Norgestrel, Nortriptylin, Nystatin, Ofloxacin, Omeprazol, Ondansetron, Pancreatin, Panthenol, Pantothensäure, Paracetamol, Penicillin G, Penecillin V, Phenobarbital, Phenoxifyllin, Phenoxymethylnpenicillin, Phenylephrin, Phenylpropanolamin, Phenytoin, Piroxicam, Polymyxin B, Povidon-Iod, Pravastatin, Prazepam, Prazosin, Prednisolon, Prednison, Bromocriptin, Propafenon, Propranolol, Proxyphyllin, Pseudoephedrin, Pyridoxin, Quinidin, Ramipril, Ranitidin, Reserpin, Retinol, Riboflavin, Rifampicin, Rutosid, Saccharin, Salbutamol, Salcatonin, Salicylsäure, Simvastatin, Somatropin, Sotalol, Spironolacton, Sucralfat, Sulbactam, Sulfamethoxyzol, Sulfasalazin, Sulpirid, Tamoxifen, Tegafur, Teprenon, Terazosin, Terbutalin, Terfenadin, Tetracyclin, Theophyllin, Thiamin, Ticlopidin, Timolol, Tranexamsäure, Tretinoin, Triamcinolon-Acetonid, Triamteren, Trimethoprim, Troxerutin, Uracil, Valproinsäure, Vancomycin, Verapamil, Vitamin E, Volinsäure, Zidovudin, Zotepin.

Wirkstoffe im Sinne der Erfindung sind auch Vitamine und Mineralstoffe, sowie Pflanzenbehandlungsmittel und Insektizide. Zu den Vitaminen gehören die Vitamine der A-Gruppe, der B-Gruppe, worunter neben B₁, B₂, B₆ und B₁₂ sowie Nicotinsäure und Nicotinamid auch Verbindungen mit Vitamin-B-Eigenschaften verstanden werden, wie z.B. Adenin, Cholin, Pantothensäure, Biotin, Adenylsäure, Folsäure, Orotsäure, Pangamsäure, Carnitin, p-Aminobenzoesäure, myo-Inosit und Liponsäure sowie Vitamin C, Vitamine der D-Gruppe, E-Gruppe, F-Gruppe, H-Gruppe, I- und J-Gruppe, K-Gruppe und P-Gruppe. Zu Wirkstoffen im Sinne der Erfindung gehören auch Peptidtherapeutika.

Der mindestens eine Wirkstoff ist in der Matrix in Form einer festen Lösung enthalten.

Der Begriff "feste Lösungen" ist dem Fachmann geläufig, beispielsweise aus "Chiou und Riegelman, J. Pharm. Sci. 60, 1281-1302 (1971). In festen Lösungen liegt der Wirkstoff molekulardispers in der Matrix vor.

Der Wirkstoffgehalt pro Dosiseinheit und die Konzentration können je nach Wirksamkeit und Freisetzungsgeschwindigkeit in weiten Grenzen variieren. Die einzige Bedingung ist, daß sie zur Erzielung der gewünschten Wirkung ausreichen. So kann die Wirkstoffkombination im Bereich von 0,1 bis 90, vorzugsweise von 0,5 bis 60 Gew.-% liegen. Gleichfalls gelten diese Angaben auch für den Bereich von Nahrungsergänzungsmitteln wie z.B. Vitaminpräparaten.

Die Herstellung der erfindungsgemäßen Zubereitungsformen kann wie im folgenden geschildert erfolgen:

Zunächst erfolgt die Überführung der Matrixkomponenten in eine plastische Masse. Dabei kann entweder eine Vormischung aller Komponenten plastifiziert werden, oder man erweicht zuerst das polymere Bindemittel und fügt dann die anderen Komponenten, also Wirkstoffe und/oder weitere Hilfsstoffe, hinzu. Die Plastifizierung erfolgt durch Energieeintrag. Je nach Zusammensetzung der Matrix erweichen die Komponenten im Bereich von 40 bis 190°C, vorzugsweise 50 bis 150°C. Welcher Temperaturbereich jeweils geeignet ist, hängt von der Glasübergangstemperatur der polymeren Bindemittel, den Eigenschaften der gegebenenfalls zugesetzten Wirkstoffe und gegebenenfalls zugesetzten Weichmachern ab. Vorzugsweise arbeitet man in Abwesenheit von Lösungsmitteln wie Wasser oder organische Lösungsmittel. Es kann sich aber auch empfehlen, geringe Mengen Wasser als Weichmacher zuzusetzen. Der optimale Temperaturbereich läßt sich durch einige einfache Handversuche ermitteln. Die Mischung der Matrixkomponenten soll so erweichen, daß die entsprechende plastische Masse eine spezifische Viskosität von 0,0007 bis 10.000 Pa·s, vorzugsweise 0,001 bis 3000 Pa.s aufweist (bei 150°C).

Der Erweichungsvorgang kann in einem Extruder, einem Kneter oder einem Mischreaktor erfolgen, wobei die plastische Masse durch Längs- und Quervermischung homogenisiert wird.

Vorzugsweise erfolgt die Plastifizierung in einem Extruder, der eine oder mehrere Schnecken aufweist, die gleich- oder gegensinnig drehen können, insbesondere in einem Zweischneckenextruder. Dieser kann mit oder ohne Knetelemente betrieben werden, wobei die Verwendung von Knetelementen wegen der besseren Durchmischung jedoch bevorzugt ist.

Während des Plastifizierungsvorgangs wird die partikuläre Phase zugegeben. Dies kann bei Verwendung eines Extruders in der heißen Zone (hot feed) oder in der kalten Zone erfolgen. Vorzugsweise werden die Partikel über Differentialdosierwaagen kontinuierlich zugeführt.

Bei welchen Temperaturen die Zugabe der partikulären Phase erfolgt, richtet sich nach der Art der Wirkstoffe und der Art der gewünschten Freisetzungsprofile. Setzt man beispielsweise Pellets zu, so kann es erwünscht sein, daß diese an der Oberfläche anschmelzen, weil dies zu einer Vergrößerung der Oberfläche führt, wodurch das Einsetzen des Diffusionsvorgangs nach Verabreichung erleichtert wird. Andererseits soll die Temperatur aber so niedrig sein, daß separate Phasen erhalten bleiben. Die gewünschte Temperatur kann durch einige einfache Versuche ermittelt werden.

Nach dem Einbetten der partikulären Phase in die Matrixphase erfolgt die Formgebung der noch plastischen Masse zu den gewünschten Darreichungsformen.

Will man beispielsweise die partikuläre Phase in eine kristalline Zuckermatrix einbetten, so empfiehlt es sich, die plastische Masse vor der Formgebung unter Rühren so weit abzukühlen, daß sie noch formbar ist, aber nach Extrusion keine abgeschreckte amorphe, glasartige Schmelze entsteht.

Die noch plastische Masse kann durch eine Düse oder Lochplatte extrudiert werden, und dann in an sich bekannter Weise zu Tabletten, Kautabletten, Lutschtabletten, Buccaltabletten, Sublingualtabletten, Kaumassen wie Kaugummis oder Zäpfchen geformt oder in Kapseln gefüllt werden. Die Formgebung erfolgt vorzugsweise durch Kalandrierung oder durch Spritzguß. Arbeitet man nach dem bekannten Verfahren der Coextrusion, lassen sich auch Mehrschichttabletten herstellen. Auch nach dem Spritzgußverfahren lassen sich Mehrschichttabletten herstellen.

Mit Hilfe des erfindungsgemäßen Verfahrens lassen sich auf einfache Weise Darreichungsformen mit sehr differenzierten Freisetzungsprofilen herstellen.

Die Zubereitungen sind mindestens zweiphasig, können aber auch mehrere Phasen enthalten, wenn man unterschiedliche partikuläre Phasen verwendet.

So kann man beispielsweise untereinander unverträgliche Wirkstoffe jeweils als getrennte partikuläre Phasen einarbeiten. Die umgebende Matrix erfüllt in diesem Fall die Funktion einer Hülle.

Bei Wirkstoffen mit stark unterschiedlichen physikalischen Eigenschaften kann man jeweils optimale partikuläre Formulierungen ebenfalls auf einfache Weise in eine Darreichungsform einarbeiten.

Besonders vorteilhaft lassen sich Darreichungsformen mit stufenförmigen Freisetzungsprofilen herstellen. So kann man beispielsweise retardierte Granulate oder Pellets eines Wirkstoffs in eine Instant-Release-Matrix, besonders günstig in eine feste Lösung, die ebenfalls diesen Wirkstoff enthält, einbetten. Auf diese Weise kann man ein schnelles Anfluten und ein lang anhaltendes plateauförmiges Freisetzungsprofil mit einer Darreichungsform erzielen. Durch Verwendung von Granulaten ein und desselben Wirkstoffs, aber mit unterschiedlichem Auflösungsverhalten, lassen sich beliebige Folgeprofile einstellen. Man kann auch Profile mit zeitverzögertem Einsatz der Wirkstoffabgabe und Wiederholungsprofile einstellen.

Gegenüber herkömmlichen durch Kompression erhaltenen Formen weisen die aus plastifizierten Massen erhaltenen Formen eine bessere Homogenität und bei Herstellung fester Formen eine geringere Porosität und bessere mechanische Stabilität auf.

### Beispiele

### Allgemeine Vorschrift

Die Herstellung der erfindungsgemäßen Zubereitungsformen erfolgte in einem Doppelschneckenextruder (ZSK-40, Firma Werner & Pfleiderer, Stuttgart). Der Extruder bestand aus insgesamt fünf separat temperierbaren Schüssen. Der letzte Schuß direkt vor der Düse war mit einer nach oben offenen Einfüllöffnung versehen, durch die Material in die bereits plastifizierte Schmelze gegeben wurde. Die Schmelze wurde dann durch eine 14 cm breite Breitschlitzdüse in Form eines Schmelzebandes ausgetragen und direkt zwischen zwei gegenläufig drehende Kalanderformwalzen geführt. Diese Formwalzen trugen auf ihrer Oberfläche Vertiefungen in Form von Tablettenhälften, so daß das Schmelzeband zu einem Band aus Tabletten verformt wurde. Der Durchsatz des Extruders betrug 20 kg/h. Die Drehzahl der Schnecken lag im Bereich von 80 bis 130 Umdrehungen pro Minute.

### Beispiel 1 (nicht erfindungsgemäß)

Eine Mischung aus 69 Gew.-% Polyvinylpyrrolidon mit einem K-Wert von 30, 30 Gew.-% Ibuprofen und 1 Gew.-% hochdisperses Kieselgel (Aerosil® 200) wurde bei Temperaturen von 60 bis 80°C im Extruder plastifiziert. Über die Einfüllöffnung kurz vor der Austrittsdüse wurden 6 kg/h an reinem kristallinem Paracetamol zudosiert. Ohne Zugabe von Paracetamol war die Schmelze klar und optisch transparent. Durch die Zugabe des kristallinen Paracetamols, das sich in der Schmelze nicht löste, wurde die Schmelze trüb. Die Schmelze wurde in dem nachgeschalteten Formbandkalander zu Oblongtabletten mit einem mittleren Tablettengewicht von 600 mg verformt.

### Beispiel 2

Analog Beispiel 1 wurden Oblongtabletten hergestellt. Statt reinem kristallinem Paracetamol wurde jedoch kristallines Paracetamol verwendet, welches zuvor durch Behandlung mit einer isopropanolischen Lösung von Ethylcellulose mit einem Filmüberzug versehen worden war.

### Beispiel 3

Eine Mischung aus 25 Gew.-% Ascorbinsäure, 8 Gew.-% Tocopherol-Acetat (TPSD 50, Fa. BASF, 50 gew.-%ige Formulierung mit einer Lactose/Caseinat-Matrix), 30 Gew.-% Hydroxypropylcellulose mit einem mw von 80.000 Dalton (Klucel® EF, Fa. Aqualon), 14,4 Gew.-% Isomalt F (Fa. Palatinit, Mannheim), 1 Gew.-% Lecithin, 1 Gew.-% Orangenaroma und 0,6 Gew.-% Aspartam wurde bei 100 bis 120°C extrudiert. Durch die Einfüllöffnung vor der Düse wurden 4 kg/h an Betacarotin-Pellets mit Korngrößen im Bereich von 100 bis 900 µ (Betavit®, Fa. BASF, stärkegepuderte, 10 gew.-%ige Betacarotin-Formulierung mit einer Gelatine/Lactose-Matrix) zudosiert. Die Schmelze wurde nach Extrusion im Formbandkalander zu Oblongtabletten mit einem mittleren Tablettengewicht von 1000 mg verformt. Die rotgefärbten Betacarotin-Pellets waren in der Tablette als diskrete Einzelpartikel zu erkennen.

## Patentansprüche

1. Verfahren zur Herstellung einer festen oder halbfesten, mindestens zweiphasigen wirkstoffhaltigen Zubereitungsform, wobei eine Phase eine Matrix bildet, die mindesten einen Wirkstoff in Form einer festen Lösung enthält, und mindestens eine weitere Phase multipartikulär in Form von Partikeln, die mindestens einen Wirkstoff und ein Additiv umfassen, in die Matrix eingebettet ist, wobei die Matrix eine Komponente umfasst, die ausgewählt ist unter unvernetzten Homo- oder Copolymeren des N-Vinylpyrrolidons mit K-Werten nach Fickentscher von 12 bis 100, Hydroxyalkylcellulosen, Celluloseestern, Acrylat- oder Methacrylat-haltigen Polymeren und Polyethylenglykolen, wobei die Polyethylenglykole ein Molekulargewicht im Bereich von 1000 bis 20 000 aufweisen, wobei man eine Mischung der Komponenten der Matrixphase durch Energieeintrag plastifiziert, während oder nach der Plastifizierung die mindestens eine weitere wirkstoff- und additivhaltige Phase in Form von Partikeln mit einer Größe von 0,5 bis 3 mm homogen in die Matrixphase einbettet und die noch plastischen Masse anschließend formt.

2. Verfahren nach Anspruch 1, **dadurch gekennzeichnet, dass** man die weitere Phase in Form von Kristallen, Pellets, Mikrotabletten oder Granulaten in die Schmelze der Matrixphase einbringt.

3. Verfahren nach einem der Ansprüche 1 oder 2, wobei man die Mischung der Matrixphase bis zu einer spezifischen Viskosität von 0,001 bis 3000 Pa·s (bei 150°C) plastifiziert.

4. Feste oder halbfeste, mindestens zweiphasige, wirkstoffhaltige Zubereitungsform, wobei eine Phase eine Matrix bildet, die mindestens einen Wirkstoff in Form einer festen Lösung enthält, und mindestens eine weitere additivhaltige Phase multipartikulär in Form von Partikeln, die mindestens einen Wirkstoff umfassen, in die Matrix eingebettet ist, erhältlich nach dem Verfahren gemäß einem der Ansprüche 1 bis 3.

## Claims

1. A process for producing a solid or semisolid, at least two-phase active ingredient-containing formulation, where one phase forms a matrix which contains at least one active ingredient in the form of a solid solution, and at least one further phase in the form of particles which comprise at least one active ingredient and an additive, is embedded into the matrix, where the matrix comprises a component which is selected from uncrosslinked homo- or copolymers of N-vinylpyrrolidone with Fickentscher K values of from 12 to 100, hydroxyalkylcelluloses, cellulose esters, acrylate- or methacrylate-containing polymers and polyethylene glycols, where the polyethylene glycols have a molecular weight in the range from 1 000 to 20 000, where a mixture of the components of the matrix phase is plasticized by energy input, at least one other active ingredient- and additive-containing phase is incorporated homogeneously in the form of particles having a size of from 0.5 to 3 mm into the matrix phase during or after the plasticization, and the material phase is subsequently shaped while still plastic.

2. A process as claimed in claim 1, where the further phase is introduced in the form of crystals, pellets, microtablets or granules into the melt of the matrix phase.

3. A process as claimed in either of claims 1 or 2, where the mixture of the matrix phase is plasticized until the specific viscosity is from 0.001 to 3 000 Pa·s (at 150°C).

4. A solid or semisolid, at least two-phase active ingredient-containing formulation, where one phase forms a matrix which contains at least one active ingredient in the form of a solid solution, and at least one further additive-containing phase in the form of particles which comprise at least one active ingredient, is embedded into the matrix, obtainable by the process as claimed in any of claims 1 to 3.

## Revendications

1. Procédé de fabrication d'une préparation solide ou semi-solide, au moins diphasique, contenant un agent actif, où une phase forme une matrice contenant au moins un agent actif sous la forme d'une solution solide, et au moins une autre phase multiparticulaire sous la forme de particules qui comprennent au moins un agent actif et un additif, est contenue dans la matrice, la matrice comprenant un composant choisi parmi les homopolymères ou les copolymères non réticulés de la N-vinylpyrrolidone avec des valeurs K selon Fickentscher de 12 à 100, les hydroxyalkylcellusoses, les esters cellulosiques, les Polymères contenant un acrylate ou un méthacrylate et les polyéthylène-glycols, les polyéthylène-glycols présentant un poids moléculaire de l'ordre de 1000 à 20000, dans lequel on plastifie un mélange des composants de la phase matricielle par apport d'énergie, on introduit, pendant ou après la plastification, au moins l'autre phase contenant un agent actif et un additif sous la forme de particules d'une taille de 0,5 à 3 mm de façon homogène dans la phase matricielle et on forme ensuite la masse encore plastique.

2. Procédé selon la revendication 1, **caractérisé en ce que** l'on introduit l'autre phase sous forme de cristaux, de pastilles, de micro-comprimés ou de granulés dans la masse fondue de la phase matricielle.

3. Procédé selon l'une quelconque des revendications 1 ou 2, dans lequel on plastifie le mélange de la phase matricielle jusqu'à obtenir une viscosité spécifique de 0,001 à 3000 Pa·s (à 150°C).

4. Préparation solide ou semi-solide, au moins diphasique, contenant un agent actif, où une phase forme une matrice qui contient au moins un agent actif sous la forme d'une solution solide, et au moins une autre phase contenant un additif, multiparticulaire, sous forme de particules, comprenant au moins un agent actif, est contenue dans la matrice, pouvant être obtenue d'après le procédé selon l'une quelconque des revendications 1 à 3.
